# EUROPÄISCHE PATENTANMELDUNG

(11) **EP 2 405 251 A2**
(43) Veröffentlichungstag der Anmeldung: **11.01.2012**
(21) Anmeldenummer: 11172858.0
(22) Anmeldetag: 06.07.2011
(51) Int. Cl.: G01N 1/20, G01N 33/34

(54) **Probenentnahme**

(30) Priorität: 08.07.2010 DE 102010031097
(71) Anmelder: Voith Patent GmbH, 89522 Heidenheim (DE)
(72) Erfinder: Leiss, Horst, 74861 Neudenau (DE); Pflügel, Reinhold, 89561 Dischingen (DE); Strempfl, Christoph, 89542 Herbrechtingen (DE)

(57) **Zusammenfassung**

Die Erfindung betrifft ein Verfahren zur Entnahme einer repräsentativen Probe während des Betriebes, insbesondere von Suspensionsproben wie sie bei der Faserstoffbahnherstellung vorkommen, aus einem Rohrleitungssystem, wobei das Rohrleitungssystem über ein Auslassventil (2) verfügt, mit folgenden Schritten:
- verbinden des Auslassventils (2) mit einer Entnahmevorrichtung (3),
- verbinden der Entnahmevorrichtung (3) mit dem Probengefäß (6), zum abfüllen der Probe
- öffnen des Auslassventils (2).

## Beschreibung

Die Erfindung betrifft ein Verfahren zur Entnahme einer repräsentativen Probe während des Betriebes, insbesondere von Suspensionsproben wie sie bei der Faserstoffbahnherstellung vorkommen, aus einem Rohrleitungssystem.

Die in der Erfindung dargelegte Lösung bezieht sich auf die Problematik, dass in der Papierindustrie zur Bestimmung verschiedener Kenngrößen, z.B. Informationen über den Trockenmasse- oder Gewichtsanteil des Feststoffanteils einer Suspension, benötigt werden.

Dazu wird während des Betriebs die Suspension über ein Auslassventil direkt aus der Rohrleitung entnommen. In der Praxis ist es für die Entnahme einer repräsentativen Probe notwendig, das Auslassventil vollständig zu öffnen, wobei es bei voll geöffnetem Auslassventil nur möglich ist große Probenmengen zu entnehmen.

Die Stoffdichte der Suspension im Rohrsystem beträgt üblicherweise zwischen ca. 0,03 und 6,0 % und die für die Messung benötigte Suspensionsprobenmenge sollte vorzugsweise nur 0,1l bis 1l betragen.

Eine saubere Entnahme derart kleiner Suspensionsmengen ist bei den vorherrschenden Drücken im Rohrleitungssystem aber nur möglich, wenn das Ventil nicht vollständig geöffnet wird. Wird das Ventil aber nur teilweise geöffnet, ist die Probe, wegen der am Ventil stattfindenden Separationsprozesse bzw. Entwässerung der Suspension, nicht repräsentativ.

In der Praxis wird in der Regel eine große Mengen Suspension, ca. 5l, aus dem freien Strahl bei möglichst weit geöffnetem Auslassventil mit einem Behältnis aufgefangen.

Im Labor wird anschließend eine Suspensionteilmenge der aus dem Behältnis entnommen, entwässert und getrocknet. Aus dem Gewichtsverhältnis der getrockneten Probe und der "nassen" Probe wird dann der Trockenanteil berechnet.

Es zeigt sich, dass dadurch die Genauigkeit sowohl bei niedrigen, z.B. 0,4% Feststoffanteil, als auch höheren Stoffdichten, z.B.> 4% Feststoffanteil, zu wünschen übrig lässt.

Die Verkettung der Fehlerquellen:
- Toleranz bei Entnahme
- Fehler beim Portionieren
führt dazu, dass es zu drastisch abweichenden Messwerten kommt (>5%), die auch mit der üblichen "Doppelbestimmungen" nicht behebbar sind.

Es ist deshalb die Aufgabe der Erfindung ein Verfahren sowie eine Vorrichtung aufzuzeigen, das/die zur Entnahme eine repräsentativen Proben aus einem Rohrleitungssystem, insbesondere von Suspensionsproben wie sie bei der Faserstoffbahnherstellung vorkommen, während des Betriebes geeignet ist und eine genau Messung ermöglicht.

Die Aufgabe wird mittels des Verfahrens nach Anspruch 1, sowie mit der Vorrichtung mit den Merkmalen des Anspruches 8 gelöst.

Erfindungsgemäß wird ein Verfahren zur Entnahme repräsentativer Proben während des Betriebes vorgeschlagen, bei dem das Auslassventil einer Rohrleitung mit einer Entnahmevorrichtung verbunden wird, welche wiederum mit einem Probengefäß verbunden wird, wobei anschließend zur Befüllung des Probengefäßes das Auslassventil geöffnet wird.

Zur weiteren Verbesserung des Messergebnisses wird vorgeschlagen das Probengefäß erst nach dem Ablassen von 0,05l bis 10l, vorzugsweise ,01l bis 1l, Suspension mit der Entnahmevorrichtung zu verbinden. Dadurch wird sichergestellt, dass keine Ablagerungen aus dem Auslassventil mit in das Probengefäß gelangen.

Das Auslassventil wird zur Probenentnahme erfindungsgemäß vorzugweise ganz geöffnet, um eine Separation der Suspension zu verhindern und dadurch ein verfälschtes Ergebnis zu erhalten.

Es ist aber auch durchaus denkbar, das Auslassventil für eine Probenentnahme immer mit einer bestimmten Ventilöffnung zwischen 10% und 90% zu öffnen, um gezielt kleiner Mengen zu entnehmen zu können.

In der Entnahmevorrichtung kann zudem, mit entsprechenden Mitteln, eine Druckreduzierung auf ein Druckniveau, das >=0,9 bar unter dem der Zapfstelle liegt oder auf Umgebungsdruck, erfolgen.

Vorzugsweise werden mit der Entnahmevorrichtung Proben von 0,5l bis 1l aus dem Rohleitungssystem im Probengefäß abgefüllt oder alternativ Suspensionsvolumen die 1g bis 8g, vorzugsweise 2g bis 3g, Trockensubstanz enthalten. So entfällt die Notwendigkeit der Entnahme einer Teilmenge für die eigentliche Messung, was im Stand der Technik zu Messungenauigkeiten führt.

Des Weiteren wird vorgeschlagen den Suspensionsstrahl nach der Druckreduzierung um mehr als 30°, vorzugsweise um 90°, abzulenken.

Die Vorrichtung zur Entnahme einer repräsentativen Probe während des Betriebes aus einem Rohrleitungssystem besteht aus einer Entnahmevorrichtung, die über ein Auslassventil mit dem Rohrleitungssystem und mit einem Probenbehälter verbindbar ist, wobei die Entnahmevorrichtung eine Druckminderungsvorrichtung umfasst.

Vorzugsweise besteht die Druckminderungsvorrichtung in der Entnahmevorrichtung aus einer Blende mit einer Blendenöffnung von vorzugsweise 5 - 25mm Durchmesser, insbesondere einem Blendendurchmesser kleiner 12mm bzw. kleiner 9mm.

Eine weitere Ausgestaltung der Erfindung sieht ferner vor, dass die Entnahmevorrichtung eine Strahlablenkung umfasst, mit dem der Suspensionsstrahl ablenkbar ist. Dabei sollte der Strahl mindestens um 30°, vorzugsweise >= 90° abgelenkt werden. Hierzu ist vorzugsweise in der Entnahmevorrichtung ein rotationssymmetrisches Ablenkteil aus Metall, Keramik oder anderen verschleißfesten Materialien zu vorhanden.

Vorteile der vorliegenden Erfindung sind:
a) Bei den üblichen Drücken kann eine representative Probe aus einer Rohrleitung entnommen werden.
b) Der Einfluss des "Laboranten" kann ausgeschlossen werden.
c) Hohe Genauigkeit bei der Bestimmung der Stoffdichte.
d) Voll reproduzierbar.

Nachfolgend wird die Erfindung anhand eines Ausführungsbeispieles näher erläutert.

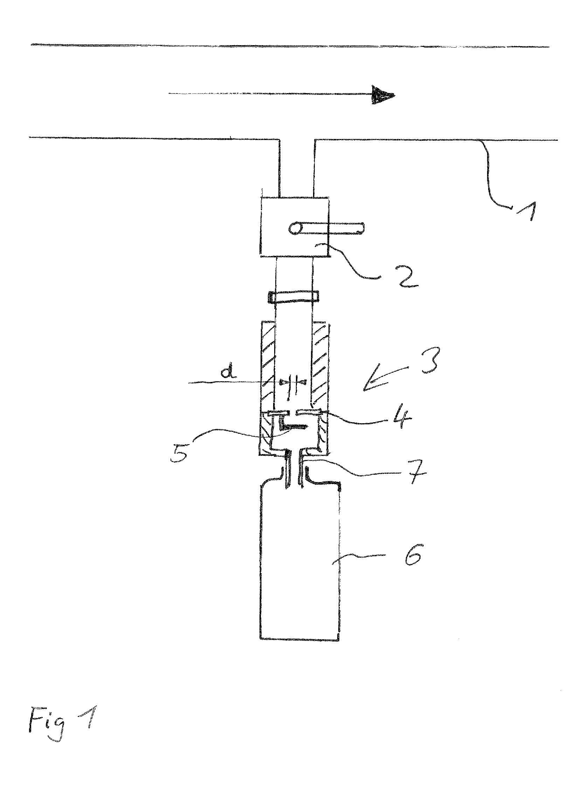

### Figur 1: Beispielhafte Ausführung einer Entnahmevorrichtung

In der Figur 1 ist beispielhaft eine Entnahmevorrichtung 3 gezeigt. Die Entnahmevorrichtung 3 ist dabei mit dem Auslassventil 2 der Rohrleitung 1 lose verbunden. Die Verbindung kann aber auch eine Schlauchverbindung oder eine andere leicht lösbare Steckverbindung sein. Das Auslassventil 2 der Rohrleitung 1 ist so ausgelegt, dass es eine Probenentnahme während des Betriebes z.B. der Papiermaschine erlaubt. Zudem ist die Geometrie derart gewählt, dass bei vollständiger Öffnung keine Separation bzw. Entwässerung der Suspension eintritt, also die Suspension keine Veränderung der Stoffdichte erlebt.

Die Entwässerungseinrichtungen bzw. Trocknungseinrichtungen, welche eine zeitnahe Bestimmung der Stoffdichte zulassen, können nur mit geringen Trockensubstanzmengen arbeiten. Typischerweise werden hierzu Probenmengen mit 2 g Trockensubstanz benötigt, da ansonst die Messung zu lange dauert, insbesondere die Zeit für die Entwässerung.

Die Entnahme einer solchen ca. 2g bis 3g Probe aus der Rohrleitung 1 mit relativ hohem Suspensionsdruck wird dadurch ermöglicht, dass in der Entnahmevorrichtung 3 eine Blende 4 mit einem Blendendurchmesser d von < 25mm (<12mm oder <9mm) vorgesehen ist, so wird sichergestellt das auch hier keine Separation bzw. Entwässerung der Suspension stattfindet oder gar die Blendenöffnung verstopft.

Zudem ist ein Strömungslenker 5 in der Entnahmevorrichtung 3 vorgesehen, der den Suspensionsstrahl um 90° ablenkt und so ein einfaches Befüllen des Probenbehälters 6 erlaubt.

Die entnommene Suspension lässt sich so gezielt entnehmen und direkt für das Messverfahren verwenden.

Die gesamte Probe, welche aus der Rohrleitung 1 entnommen wurde, wird anschließend eingewogen, entwässert, getrocknet und zur Bestimmung der verschiedenen Kenngrößen wie z.B. der Trockenmasse herangezogen.

### Bezugszeichenliste

- 1: Rohrleitung
- 2: Auslassventil
- 3: Entnahmevorrichtung
- 4: Blende
- 5: Strahlablenker
- 6: Probenbehälter
- 7: Entnahmeöffnung
- d: Blendendurchmesser

## Patentansprüche

1. Verfahren zur Entnahme einer repräsentativen Probe während des Betriebes, insbesondere von Suspensionsproben wie sie bei der Faserstoffbahnherstellung vorkommen, aus einem Rohrleitungssystem, wobei das Rohrleitungssystem über ein Auslassventil (2) verfügt, mit folgenden Schritten:
- verbinden des Auslassventils (2) mit einer Entnahmevorrichtung (3),
- verbinden der Entnahmevorrichtung (3) mit dem Probengefäß (6), zum abfüllen der Probe
- öffnen des Auslassventils (2).

2. Verfahren nach Anspruch 1
**dadurch gekennzeichnet,**
**dass** das Probengefäß (6) nach dem Ablassen von 5l bis 100l Suspension mit der Entnahmevorrichtung (3) verbunden wird.

3. Verfahren nach Anspruch 1 oder 2
**dadurch gekennzeichnet,**
**dass** das Auslassventil (2) vorzugsweise ganz geöffnet wird.

4. Verfahren nach Anspruch 1 oder 2
**dadurch gekennzeichnet,**
**dass** das Auslassventil (2) zwischen 30% und 100% geöffnet wird.

5. Verfahren nach einem der vorangehenden Ansprüche
**dadurch gekennzeichnet,**
**dass** in der Entnahmevorrichtung (3) eine Druckreduzierung auf ein Druckniveau das >=0,9 bar unter dem der Zapfstelle liegt oder auf Umgebungsdruck erfolgt.

6. Verfahren nach einem der vorangehenden Ansprüche
**dadurch gekennzeichnet,**
**dass** mit der Entnahmevorrichtung (3) Proben von 0,5l bis 1l aus dem Rohleitungssystem im Probengefäß angefüllt werden.

7. Verfahren nach einem der vorangehenden Ansprüche
**dadurch gekennzeichnet,**
**dass** mit der Entnahmevorrichtung ein Suspensionsvolumen abgenommen wird, dass 1g bis 8g, vorzugsweise 2g bis 3g, Trockensubstanz enthält.

8. Vorrichtung zur Entnahme einer repräsentativen Proben während des Betriebes, insbesondere von Suspensionsproben wie sie bei der Faserstoffbahnherstellung vorkommen, aus einem Rohrleitungssystem, wobei das Rohleitungssystem über ein Auslassventil (2) verfügt, welches mit einer Entnahmevorrichtung (3) verbindbar ist
**dadurch gekennzeichnet,**
**dass** die Entnahmevorrichtung (3) Mittel umfasst, die eine Druckminderung bewirken.

9. Vorrichtung nach Anspruch 8
**dadurch gekennzeichnet,**
**dass** zur Druckminderung eine Blende (4) verwendet wird, wobei die Blende (4) einen Blendendurchmesser (d) von kleiner 25mm, vorzugsweise kleiner 12mm bzw. kleiner 9mm aufweißt.

10. Vorrichtung nach Anspruch 9
**dadurch gekennzeichnet,**
**dass** die Entnahmevorrichtung (3) eine Strahlumlenkung (5) umfasst, die den Suspensionsstrahl nach dem Durchtritt durch die Blende (4) um mindestens 30° vorzugsweise um >=90° umlenkt.
